# EUROPEAN PATENT SPECIFICATION

(11) **EP 3 870 284 B1**
(45) Date of publication and mention of the grant of the patent: **30.07.2025**
(21) Application number: 19934887.1
(22) Date of filing: 27.06.2019
(51) Int. Cl.: A61N 5/10, A61N 5/01, A61N 5/04, A61N 5/06, A61N 5/067, A61N 5/073, A61N 5/08

(54) **AN ADJUSTABLE THERAPEUTIC FACE MASK**
JUSTIERBARE THERAPEUTISCHE GESICHTSMASKE
MASQUE FACIAL THÉRAPEUTIQUE RÉGLABLE

(43) Date of publication of application: 01.09.2021
(73) Proprietor: Shiseido Americas Corporation, New York, NY 10017 (US)
(72) Inventor: Gross, Dennis F., New York, NY 10022 (US)
(74) Representative: Valet Patent Services Limited
(86) International application number: PCT/US2019/039444
(87) International publication number: WO 2020/263258

(56) References cited:
- WO-A1-2017/127368
- KR-A- 20110 117 481
- KR-A- 20190 027 491
- KR-B1- 101 497 619
- TW-A- 201 204 425
- US-A- 4 996 981
- US-A1- 2004 162 549
- US-A1- 2014 350 643
- US-A1- 2016 056 653
- US-A1- 2016 158 569

## Description

### FIELD OF THE INVENTION

The present invention relates to a therapeutic device. More particularly, the present invention relates to a therapeutic full face light therapy mask for providing light therapy to a user's skin. A further and more specific aspect of the present invention relates to a therapeutic face mask having a plurality of light emitting diodes (LEDs) for providing multi -wavelength light spectrum with various intensities to a user's skin.

### BACKGROUND OF THE INVENTION

Various devices have been known in the prior art that are used for skin therapy, skin rejuvenation, and other beauty treatment purposes. These devices are able to provide significantly enhanced biological and/or therapeutic results. Accordingly, the use of light therapy to enhance the characteristics of the skin is widely accepted. However, various devices available for applying electrical energy to the skin are having significant structural and functional shortcomings.

Presently, most of the known light therapy devices are providing light energy to the skin via light therapy tubes that engage with the surface of the user's skin, either directly or indirectly. This process can be quite uncomfortable for a user in case of a light tube directly contacting the user's skin and can result in irritation of the skin after prolonged contact. The therapeutic benefits of the aforementioned devices take place substantially within the area where the light tubes are in contact with the skin. Further, the devices which provide indirect contact of the light tube with the skin via an intermediate layer in between are not able to disperse the desired light intensity due to loss of light in the intermediate layer. As a result, beneficial light therapy becomes unevenly distributed and ineffective to the user.

There are some light therapy devices which are not having battery thereby requiring an electrical connector to be coupled with an external electric power supply which is not possible always.

The light therapy face mask known in the prior art do not enable the face mask to have communication with an external device in order to perform a plurality of functions.

The light therapy face mask products which are available in the market are of fixed size and are hand-held type devices. These hand-held type face masks have proven quite less satisfactory in providing the best or desired light dispersion. The alternative is a customer visiting a doctor to receive light treatment which is neither convenient nor affordable.

A hands-free therapeutic experience is always better than the device to be held in a particular position for extended periods of time during the therapy. Numerous assemblies have been conceived for mounting face mask devices by varieties of straps, bands, wraps, and cords which can result in a pressing of the support and mounting assembly closely against the head or back side neck area of a patient. There is always a need to minimize the extent of such mounting and supporting assemblies in order to make their impact on the patient thereby enhancing the patient's comfort during the therapy. Customers always rely on those face mask light therapy products which are light-weight and which require minimal supporting means during the therapeutic usage. As a user's face comes in different shape and size, the face mask should also be able to adjust according to the user's face shape and size in order to have an efficient therapeutic application of the face mask on the desired area on the face of the user. Additionally, the currently available devices for facial treatment are using separate patches which must rest on the eye area of a user so as to block the light from the light tubes inside the mask. Some of the solutions in this regard are listed below:
KR 2019 0027491 A discloses an electrical stimulation device that is also capable of providing light therapy. A cosmetic mask entirely covers the face, however including cut-outs for eyes, nose and mouth portions in one embodiment and an eyeglass shoe to protect the eyes in another embodiment. A convex lens in an eye spectacle is used to irradiate eyes to relieve eye fatigue.
TW 201 204 425 A discloses a face mask capable of providing light therapy and heating effects on the face of the user. Further a control unit controls the provision of light therapy and heating effects. Inputs to the control unit are received from push buttons or a touchscreen.
KR 101 497 619 B1 discloses a multi-layer face mask with several LEDs to provide light therapy. Further a tubular shaped cover, with flanges protruding on both sides, has been provided to prevent eyes from irradiation and foreign material. Therefore, during the provision of light therapy, the eyes remain covered by the tubular shaped protector.
KR 2011 0117481 A discloses another face mask for providing light therapy through multiple LEDs. The light from the LEDs is diffused using a silicone layer. To protect the eyes from irradiation, protrusion pads are proposed that may be integrally formed with outer cover or may be coupled to the outer cover. Here again eyes of the user remain covered during the light therapy.
US 2016/056653 A1 discloses a phototherapy lamp which can charged from a cellular phone. The lamp includes several LEDs to provide light therapy to the face of the user, In one embodiment, goggles or an eyeglass frame to shield the eyes of the user from lamp radiation. In another embodiment, illustrated in Fig. 18 of D5, the LEDs are arranged to provide phototherapy to the eyes of the user, however, eyeglass frame or goggles are still maintained in the embodiment.
US 2016/158569 A1 discloses a facial treatment device and a system including the treatment device combining light and electromagnetic therapy for facial treatments. The facial treatment device includes a mask base; a plurality of light emitting diodes (LEDs) disposed across an inner side of the facial treatment device; and one or more electromagnetic field producing units, each configured for producing an electromagnetic field located over the periphery of the mask base. The system includes the facial treatment device and a control unit that allows controlling the operation of the LEDs and the one or more electromagnetic field producing units.
WO 2017/127368 A1 discloses a pad having a circuit board and multiple light sources to emit light having a wavelength in a range of 300 nanometers (nm) to 1100 nm for light therapy treatment of a human. A front exterior layer may have multiple raised portions to cover the multiple light sources, respectively, the front exterior layer to face a body part of the human in treatment. The pad may be conformable to the body part.

Therefore, there is a long felt need for an improved face mask for light therapy that can be readily adaptable to communicate therapeutic light to areas near the eyes thereby preventing any possible causation of damage to the eyes of a patient.

### OBJECTS OF THE PRESENT INVENTION

It would be highly advantageous, therefore, to alleviate the shortcomings and the deficiencies inherent in the prior art. Few of the objects of the present invention are listed below:
▪ It is an object of the present invention to provide a therapeutic face mask which provides light therapy to a plurality of disorders of the facial skin of a user;
▪ It is another object of the present invention to provide a therapeutic face mask that is easy to use;
▪ It is a further object of the present invention to provide a therapeutic face mask that can be placed on a user's face and that can be made to disperse different colors and intensities of light based on the user's input;
▪ It is an additional object of the present invention to provide a therapeutic face mask which prevents the direct contact of the light tubes with the user's skin;
▪ It is a further object of the present invention to provide a therapeutic face mask that is comfortable to use and does not require to be held in a particular position when used by a user;
▪ It is still further object of the present invention to provide a therapeutic face mask that allows therapeutic light to be evenly and uniformly dispersed over the facial skin of a user;
▪ It is another object of the present invention to provide a therapeutic face mask that can be connected to an external device having a software application to control a plurality of characteristics of the light without removing the face mask from a user's face;
▪ It is yet another object of the present invention to provide a therapeutic face mask that is having an internal rechargeable power supply unit;
▪ It is a further object of the present invention to provide a therapeutic face mask which prevents the light illumination near a user eyes area to avoid light damage or irritation to a patient's or user's eyes; ▪ It is another object of the present invention to provide a therapeutic face mask which allows a user to perform another task when undergoing light therapy; and
▪ It is another object of the present invention to provide a therapeutic face mask utilizing a multi - wavelength spectrum of light with variable intensities to stimulate the development of collagen and to destroy the acne causing bacteria on the facial area of a user.

Other objects, aspects, features, and goals of the present invention will be better understood from the detailed description of the present invention.

### SUMMARY OF THE INVENTION

The invention relates to an adjustable therapeutic face mask as defined in the appended claims. Embodiments, examples or aspects in the following disclosure, in particular methods, which do not fall under the scope of the claims are presented for illustration purposes only and do not form part of the invention.

It should be understood that the present invention disclosure is not limited to particular systems and methodologies described herein, as there can be multiple possible aspects of the present invention disclosure which are not expressly illustrated herein. It is to be understood that the terminology used in the description is for the purpose of describing the particular versions or embodiments only and is not intended to limit the scope of the present invention disclosure.

According to an aspect of the present invention, an adjustable therapeutic face mask is disclosed. The adjustable therapeutic face mask comprises a mask body having an interior portion and an exterior portion, a flexible circuit board that includes a plurality of light emitting diodes (LEDs), a controlling unit and a transparent silicone material sheet.

The mask body is substantially concave shaped to accommodate a user's face and has separate cutouts for eyes, nostrils, and mouth so that the user can see and breathe normally when undergoing therapy sessions. The flexible circuit board is situated between the interior portion of the mask body and the transparent silicon material sheet. The exterior portion of the mask body has a forehead cover, right and left cheek covers and a chin cover for improving aesthetic appearance of the therapeutic mask and providing support to the mask body. The forehead cover accommodates a mounting bracket which is attached to the exterior portion of the mask body via screws thereby creating a hollow portion with the forehead cover. The hollow portion accommodates a set of rechargeable batteries and the controlling unit. A push button is protruding through the forehead cover where the user can select different colors of the lights for skin treatment. The therapeutic mask has a charging port for recharging the batteries by directly plugging-in an adapter to an external electric socket or via a universal serial bus (USB) cable or any other suitable charging medium.

According to another aspect of the present invention, the controlling unit has a communication module which allows a wired or wireless communication with an electronic communication device including but not limited to a mobile, a laptop, or computer and the like.

A software application is installed in the electronic communication device and a connection via Wi-Fi, Bluetooth, near field communication (NFC) is established with the therapeutic face mask. The software application allows the user to select the desired color, frequency, wavelength of the light without removing the mask from his/her face.

To use the therapeutic face mask, a user has to wear the face mask on his/her face by tightening the mask with a strap that is detachably attached to the forehead cover and left and right cheek covers of the therapeutic face mask. The strap is made of elastic material and can be adjusted as per the size and shape of the user's face. The user can change the color, intensity, frequency, and wavelength of the LEDs by using the buttons protruding through the forehead cover or the user can change the color, intensity, frequency, and wavelength of the light by using the software application installed in his/her electronic communication device. Once the user selects a particular color, frequency, intensity, and wavelength, the therapeutic session is initiated by the therapeutic mask. After the therapeutic session is concluded, the light emitting diodes stop emitting the light thereby making the therapeutic face mask to go OFF.

According to an additional aspect of the present invention, a user can choose any color including but not limited to red, blue, violet, white as per his requirement as each color has its own beneficial effect on the user's skin.

According to another aspect of the present invention, the flexible circuit board can have red, blue, green (RBG) LEDs in order to enhance the luminosity of the therapeutic face mask.

According to yet another aspect of the present invention, the outer edges of the transparent silicon material sheet near the eyes area of the user are made opaque through painting or through any other suitable method so as to prevent the illumination of light on the user' s eyes thereby preventing any possible effect on the normal vision of the user and allowing the user to perform his/her normal task when undergoing the therapy.

The following description is illustrative in nature and is not intended to be in any way limiting. In addition to the illustrative aspects, embodiments, and features described above, further aspects, embodiments, and features will be apparent by reference to the following detailed description in conjunction with the accompanying drawings.

### BRIEF DESCRIPTION OF THE ACCOMPANYING DRAWINGS

The accompanying drawings illustrate the best mode for carrying out the invention as presently contemplated and set forth hereinafter. The present invention may be more clearly understood from a consideration of the following detailed description of the preferred embodiments taken in conjunction with the accompanying drawings wherein like reference letters and numerals indicate the corresponding parts in various figures in the accompanying drawings, and in which:

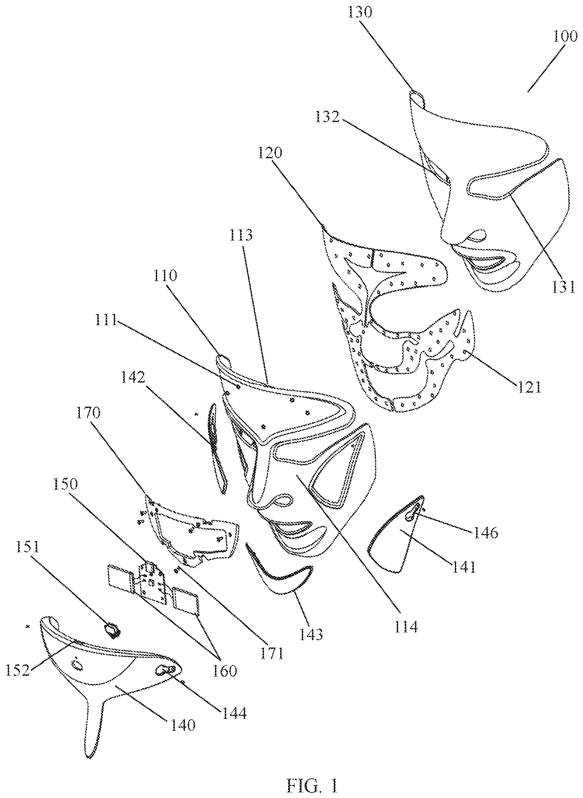

Fig. 1 illustrates an isometric view of an adjustable therapeutic face mask according to one embodiment of the present invention;

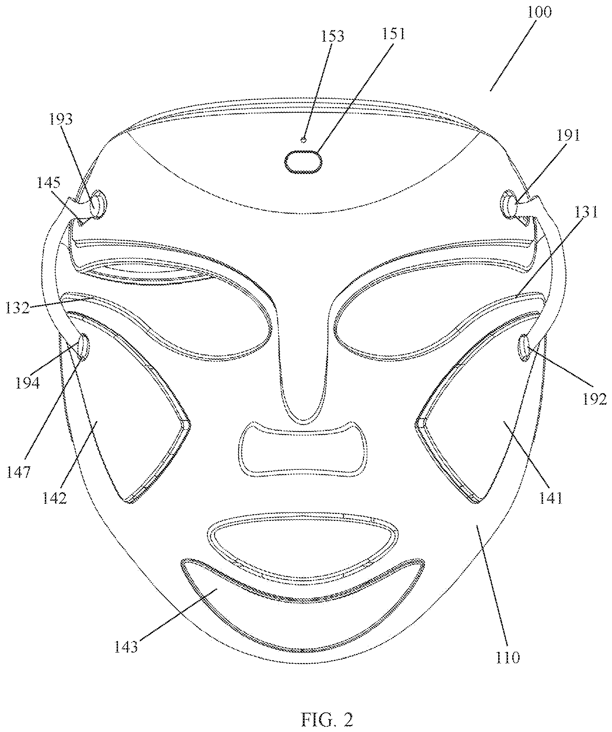

Fig. 2 illustrates a front side view of the adjustable therapeutic face mask according to one embodiment of the present invention;

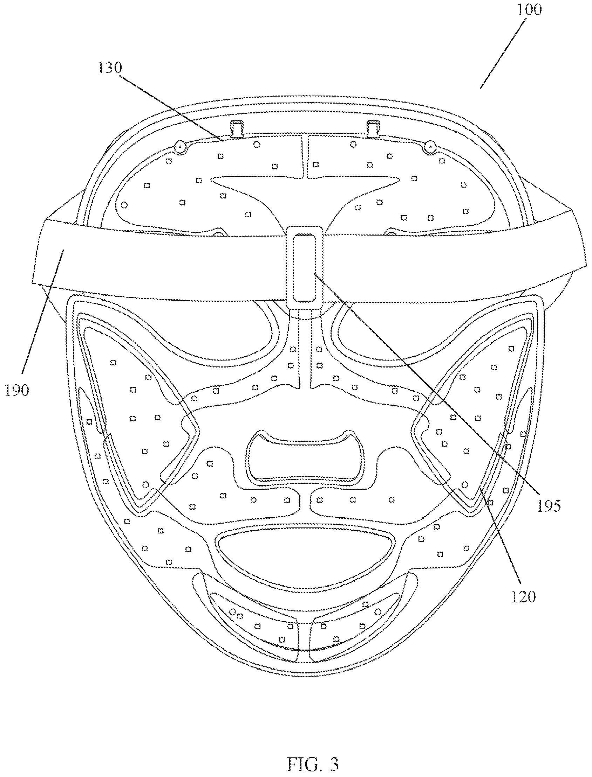

Fig. 3 illustrates a back side view of the adjustable therapeutic face mask according to one embodiment of the present invention;

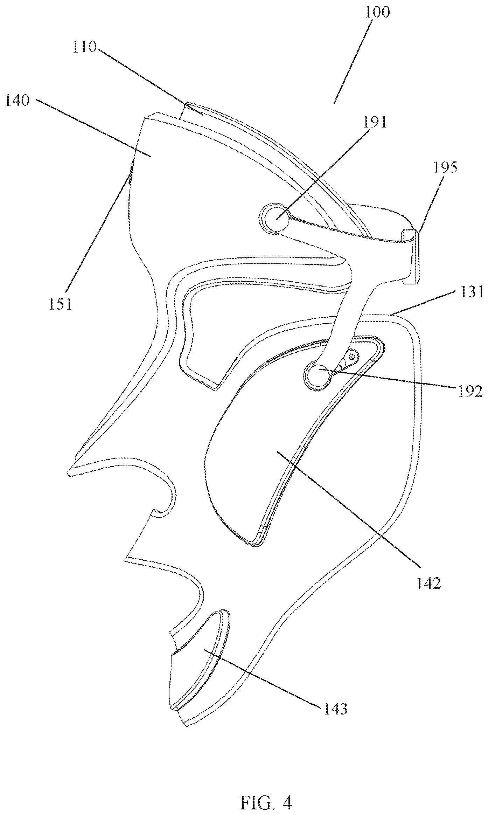

Fig. 4 illustrate a side view of the adjustable therapeutic face mask according to one embodiment of the present invention; and
Fig. 5 illustrate a top view of the adjustable therapeutic face mask according to one embodiment of the present invention.

### DETAILED DESCRIPTION OF THE PRESENT INVENTION

Embodiments of the present invention disclosure will be described more fully hereinafter with reference to the accompanying drawings in which like numerals represent like elements throughout the figures, and in which example embodiments are shown.

The detailed description and the drawings illustrate specific exemplary embodiments by which the disclosure may be practiced. These embodiments are described in detail to enable those skilled in the art to practice the disclosure. It is to be understood that other embodiments may be utilized, and other changes may be made, without departing from the scope of the present disclosure. The following detailed description is therefore not to be taken in a limiting sense, and the scope of the present invention is defined by the appended claims. Embodiments of the claims may, however, be embodied in many different forms and should not be construed as limited to the embodiments set forth herein.

Embodiments of the present disclosure are discussed below with reference to the Figures. However, those skilled in the art will readily appreciate that the detailed description given herein with respect to these figures is for explanatory purposes as the disclosure extends beyond these limited embodiments. For example, it should be appreciated that those skilled in the pertinent art will, in light of the teachings of the present disclosure recognize a multiplicity of alternate and suitable approaches depending upon the needs of the particular application to implement the functionality of any given details described herein beyond the particular implementation choices in the following embodiments described and shown. That is to say that there are numerous modifications and variations of the present disclosure that are too numerous to be listed here, but that all fit within the scope of the disclosure. Also, singular words should be read as plural and vice versa and masculine as feminine and vice versa, where appropriate, and alternative embodiments do not necessarily imply that the two are mutually exclusive.

It is to be further understood that the present disclosure is not limited to the particular methodology, compounds, materials, manufacturing techniques, uses, and applications, described herein, as these may vary. It is also to be understood that the terminology used herein is used for the purpose of describing particular embodiments only, and is not intended to limit the scope of the present disclosure. It must be noted that as used herein and in the appended claims, the singular forms "a," "an," and "the" include the plural reference unless the context clearly dictates otherwise. Thus, for example, a reference to "an element" is a reference to one or more elements and includes equivalents thereof known to those skilled in the art. Similarly, for another example, a reference to "a step" or "a means" is a reference to one or more steps or means and may include sub-steps and subservient means. All conjunctions used are to be understood in the most inclusive sense possible. Thus, the word "or" should be understood as having the definition of a logical "or" rather than that of a logical "exclusive or" unless the context clearly necessitates otherwise. Structures described herein are to be understood also to refer to functional equivalents of such structures. The language that may be construed to express approximation should be so understood unless the context clearly dictates otherwise.

Unless defined otherwise, all technical and scientific terms used herein have the same meanings as commonly understood by one of ordinary skill in the art to which this disclosure belongs. Preferred methods, techniques, devices, and materials are described, although any methods, techniques, devices, or materials similar or equivalent to those described herein may be used in the practice or testing of the present disclosure. Structures described herein are to be understood also to refer to functional equivalents of such structures. The present disclosure will now be described in detail with reference to embodiments thereof as illustrated in the accompanying drawings.

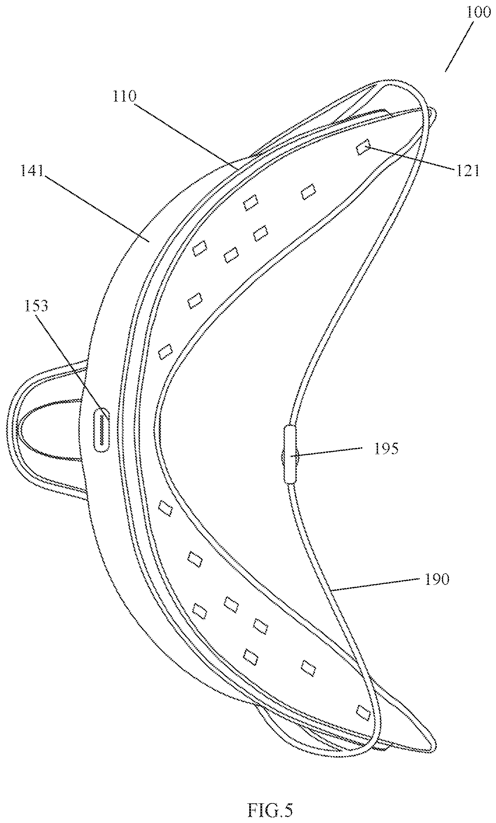

Referring now to FIG. l through FIG.5, a therapeutic face mask 100 is shown according to one embodiment of the present invention. The therapeutic face mask 100 is in a shape of the human face and have different cutouts for eyes, nostrils and mouth area to allow a user to see, breath and speak normally when using the therapeutic face mask 100 and comfortably fixing it on the user's face. The therapeutic face mask 100 is comprising a mask body 110 having an interior portion 113 and an exterior portion 114, a flexible circuit board 120 having a plurality of light emitting diodes (LEDs) 121 towards the interior portion 113 of the mask body 110, a controlling unit 150 coupled to the exterior portion 114 of the mask body, and a transparent silicone material sheet 130 covering the flexible circuit board 120 towards the interior portion 113 of the mask body 110. The mask body 110 is made of a rigid material.

The flexible circuit board 120 also has the same cutouts for eyes, nostrils, and mouth as the mask body 110 have. The flexible circuit board 120 is situated between the mask body 110 and the transparent silicone material sheet 130. Further, the light emitting diode 121 mounted on the flexible circuit board 120 are in electrical communication with a set of rechargeable batteries 160.

The exterior portion 114 of the mask body 110 is supported by a forehead cover 140, a left cheek cover 141, a right cheek cover 142, and a chin cover 143. The forehead cover 140 with the mask body 110 make a hollow recess which accommodates a mounting bracket 170 for storing the controlling unit 150 and a set of batteries 160. The forehead cover 140, left cheek cover 141, right cheek cover 142, and the chin cover 143 are also enhancing the aesthetic appearance of the face mask. The mounting bracket 170 is fixed to the mask body 110 via a plurality of screws 171 inserted in corresponding screw thread hole 111 on the mask body 110. Further, the left and the right cheek cover (141, 142) also fixed to the mask body 110 via a set of screws.

A push button 151 is protruding through the forehead cover 140 to provide input to the controlling unit 150. A notification light 153 is also protruding through the forehead cover 140 for providing notification for battery charging and battery drain. Further, a charging port 152 is also protruding through the forehead cover 140 and is in electrical communication with the set of rechargeable batteries 160.

A strap 190 is divided into four arms with two arms in the left side and two arms in the right side in order to fix the strap 190 on to the mask body 110. The two left arms have two rounded portions (191, 192) snugly fitted in a port 144 on the forehead cover 140 and a port 146 on the left cheek cover 141 respectively. Further, the two right arms also have two rounded portions (193, 194) snugly fitted in a port 145 on the forehead cover 140 and a port 147 on the right cheek cover 142 respectively. The strap 190 also has an adjuster element 195 to adjust the strap 190 when worn by the user.

The transparent silicone material sheet 130 also has cutouts for the eyes, nostrils, and mouth in order to completely align with the mask body 110. Left and right side edges near the eyes area (131, 132) of the transparent silicone material sheet 130 are made opaque to avoid light damage or irritation to a patient's eyes.

According to another embodiment of the present invention, the controlling unit 150 can have a communication module which enables a wired or wireless communication with an external device including but not limited to a mobile, a laptop, or the computer and the like.

A software application can be installed in the external device and a connection via Wi-Fi, Bluetooth, near field communication (NFC)is established with the therapeutic face mask 100. The software application allows the user to select the desired color, frequency, wavelength of the light without removing the therapeutic face mask 100 from his/her face.

According to an advantageous embodiment of the present invention, the flexible circuit board 120 can have red, blue, green (RBG) type LEDs in order to enhance the luminosity of the therapeutic face mask 100.

According to another advantageous embodiment of the present invention, the therapeutic face mask 100 is having a set of rechargeable batteries 160 allowing the user to use the therapeutic face mask 100 in the hands-free mode and on the go.

Best Mode of using the present invention: To use the therapeutic face mask 100, a user has to wear the therapeutic face mask 100 on to his/her face by tightening the therapeutic face mask 100 with a strap 190 that is detachably attached to the forehead cover 140 and left and right cheek cover (141, 142) of the therapeutic face mask 100. The strap 190 is made of elastic material and can be adjusted as per the size and shape of the user's face. The user can change the color, intensity, frequency and wavelength of the light emitting diodes 121 by using the push buttons 151 protruding through the forehead cover 140 or the user can change the color, intensity, frequency and wavelength of the light by using the software application installed in his/her external device. Once the user selects a particular color, frequency, intensity, and wavelength the therapeutic face mask 100 initiates the therapeutic session. After the session concludes, the light emitting diodes 121 stops emitting the light or the therapeutic face mask 100 goes into OFF mode.

In the most preferred embodiment of the present invention, the therapeutic face mask is a full face therapy treatment mask for alleviating the wrinkles and acne on the facial area of the user. The present therapeutic face mask device utilizes multi-wave spectrums of LED lights to stimulate collagen development wherein the multi-wave spectrum preferably consists of LED lights of wavelength 880nm, 660nm, 630nm, and 605nm. These particular wavelengths of LED light are found to be effective in destroying the acne causing bacteria particularly at the wavelength value of 630nm and 415nm. These LEDs are custom dual chip/spectrum Surface Mount Technology (SMT) Medical Grade LEDs. Further, these LEDs are embedded in a clear and transparent sheet of silicon material using thermoplastic rubber (TPR), or in thermoplastic polyurethane (TPU) or in a thermoplastic elastomer (TPE). The design of the present therapeutic mask is unique in that it incorporates the mask body made of polycarbonate skeleton with aesthetic metallic coverings on the exterior to provide a unique cosmetic design. The area treating the skin is unique in that the construction and production of the device incorporates a unique silicone injection overlay to protect the flexible printed circuit board (PCB) and light emitting diodes (LEDs) while providing extreme clarity to deliver multi - wavelength light spectra to the skin surface of the user. The present therapeutic face mask device is portable and operates on either a direct 110v/220v adapter or rechargeable battery. The present therapeutic face mask device is adapted to function in at least one functioning mode viz., either in the mode of treatment of wrinkle or in the mode of treatment of acne or a combination of both. The present therapeutic face mask device incorporates a software adapted to control the features of optical output, treatment times, operation, recharging and auto shut off features.

Certain embodiments of this invention are described herein, including the best mode known to the inventors for carrying out the invention. Of course, variations on these described embodiments will become apparent to those of ordinary skill in the art upon reading the foregoing description.

Although specific embodiments and certain structural arrangements have been illustrated and described herein, it will be clear to those skilled in the art that various other modifications and embodiments may be made incorporating the scope of the underlying inventive concepts and that the same is not limited to the particular methods and structure herein shown and described except in so far as determined by the scope of the appended claims.

## Claims

1. An adjustable therapeutic face mask (100) , wherein said adjustable therapeutic face mask (100) substantially covers a human face, comprising:
a mask body (110) having an interior portion (113) and an exterior portion (114);
a flexible circuit board (120) including a plurality of light emitting diodes (LEDs) (121) towards the interior portion (113) of said mask body (110);
a controlling unit (150) coupled to the exterior portion of said mask body (110); and
a transparent silicone material sheet (130) covering said flexible circuit board (120), wherein said plurality of LEDs (121) emit different colors and intensities of light based on inputs from said controlling unit (150), wherein said mask body (110) has cutouts for eyes, nostrils, and mouth to comfortably fix said mask (100) on to a human face, wherein said flexible circuit board (120) is fixed in between said mask body (110) and said transparent silicon material sheet (130) towards the interior portion (113) of said mask body (110);
**Characterized in that:**
the edges (131, 132) of said transparent silicone material sheet (130) near the eye area of a user are made opaque to prevent the illuminated light entering a user's eyes.

2. The adjustable therapeutic face mask (100) according to claim 1, wherein said mask body (110) is made of a rigid material.

3. The adjustable therapeutic face mask (100) according to claim 1, wherein said controlling unit (150) is configured to control the color and intensity of light from said LEDs (121) via buttons including push button (151) mounted towards the exterior portion (114) of said mask body (110).

4. The adjustable therapeutic face mask according to claim 1, wherein the controlling unit (150) and the plurality of LEDs (121) powered by a set of rechargeable batteries (160) mounted on said mask body (110).

5. The adjustable therapeutic face mask (100) according to claim 1, wherein said LEDs (121) are custom dual chip/spectrum Surface Mount Technology, SMT, Medical Grade LEDs.

6. The adjustable therapeutic face mask (100) according to claim 1, wherein a stretchable strap (190) is detachably attached to said face mask (100) for adjustably securing said face mask (100) over the facial skin area of a user.

7. An adjustable therapeutic face mask (100) according to claim 1, wherein said controlling unit (150) has a communication module adapted to enable communication between an external digital electronic communication device and said adjustable therapeutic face mask (100).

8. The adjustable therapeutic face mask (100) according to claim 7, wherein a user can adjust colors and intensities of said plurality of LEDs (121) using a software application installed in said external device without removing said face mask (100).

9. The adjustable therapeutic face mask (100) according to claim 7, wherein the face mask is adapted to utilize multi-wavelength spectrums of LED light to stimulate collagen development.

10. The adjustable therapeutic face mask (100) according to claim 9, wherein said multi-wavelength spectrum includes a light having the wavelength selected from the group consisting of 880nm, 660nm, 630nm, and 605nm.

11. The adjustable therapeutic face mask (100) according to claim 5, wherein the LEDs (121) are embedded in silicon material using thermoplastic material selected from the group consisting of a thermoplastic rubber, TPR or a thermoplastic polyurethane, TPU, or and a thermoplastic elastomer, TPE.

## Patentansprüche

1. Einstellbare therapeutische Gesichtsmaske (100), wobei die einstellbare therapeutische Gesichtsmaske (100) im Wesentlichen ein menschliches Gesicht bedeckt, umfassend:
einen Maskenkörper (110) mit einem inneren Abschnitt (113) und einem äußeren Abschnitt (114);
eine flexible Leiterplatte (120), die eine Vielzahl von Leuchtdioden (LED) (121) in Richtung des inneren Abschnitts (113) des Maskenkörpers (110) enthält;
eine Steuereinheit (150), die mit dem äußeren Abschnitt des Maskenkörpers (110) verbunden ist; und
eine transparente Silikonmaterialfolie (130), die die flexible Leiterplatte (120) bedeckt, wobei die Vielzahl von LED (121) unterschiedliche Farben und Lichtintensitäten auf der Grundlage von Eingaben von der Steuereinheit (150) emittieren, wobei der Maskenkörper (110) Ausschnitte für Augen, Nasenlöcher und Mund aufweist, um die Maske (100) bequem auf einem menschlichen Gesicht zu befestigen, wobei die flexible Leiterplatte (120) zwischen dem Maskenkörper (110) und der transparenten Silikonmaterialfolie (130) in Richtung des inneren Abschnitts (113) des Maskenkörpers (110) befestigt ist;
**dadurch gekennzeichnet, dass:**
die Ränder (131, 132) der transparenten Silikonmaterialfolie (130) in der Nähe des Augenbereichs eines Benutzers undurchsichtig gemacht werden, um zu verhindern, dass das beleuchtete Licht in die Augen eines Benutzers eindringt.

2. Einstellbare therapeutische Gesichtsmaske (100) nach Anspruch 1, wobei der Maskenkörper (110) aus einem starren Material hergestellt ist.

3. Einstellbare therapeutische Gesichtsmaske (100) nach Anspruch 1, wobei die Steuereinheit (150) so konfiguriert ist, dass sie die Farbe und Lichtintensität von den LED (121) über Knöpfe steuert, einschließlich eines Druckknopfes (151), der an dem äußeren Abschnitt (114) des Maskenkörpers (110) angebracht ist.

4. Einstellbare therapeutische Gesichtsmaske nach Anspruch 1, wobei die Steuereinheit (150) und die Vielzahl von LED (121) von einem Satz wiederaufladbarer Batterien (160) gespeist werden, die an dem Maskenkörper (110) angebracht sind.

5. Einstellbare therapeutische Gesichtsmaske (100) nach Anspruch 1, wobei die LED (121) kundenspezifische Dual-Chip/Spektrum-LED in Oberflächenmontagetechnik (SMT) mit medizinischer Qualität sind.

6. Einstellbare therapeutische Gesichtsmaske (100) nach Anspruch 1, wobei ein dehnbares Band (190) abnehmbar an der Gesichtsmaske (100) angebracht ist, um die Gesichtsmaske (100) einstellbar über dem Gesichtshautbereich eines Benutzers zu befestigen.

7. Einstellbare therapeutische Gesichtsmaske (100) nach Anspruch 1, wobei die Steuereinheit (150) ein Kommunikationsmodul aufweist, das geeignet ist, die Kommunikation zwischen einer externen digitalen elektronischen Kommunikationsvorrichtung und der einstellbaren therapeutischen Gesichtsmaske (100) zu ermöglichen.

8. Einstellbare therapeutische Gesichtsmaske (100) nach Anspruch 7, wobei ein Benutzer die Farben und Intensitäten der Vielzahl von LED (121) unter Verwendung einer in der externen Vorrichtung installierten Softwareanwendung einstellen kann, ohne die Gesichtsmaske (100) abzunehmen.

9. Einstellbare therapeutische Gesichtsmaske (100) nach Anspruch 7, wobei die Gesichtsmaske so angepasst ist, dass sie Multiwellenlängenspektren von LED-Licht verwendet, um die Kollagenentwicklung zu stimulieren.

10. Einstellbare therapeutische Gesichtsmaske (100) nach Anspruch 9, wobei das Multiwellenlängenspektrum ein Licht mit einer Wellenlänge umfasst, die aus der Gruppe ausgewählt ist, die aus 880 nm, 660 nm, 630 nm und 605 nm besteht.

11. Einstellbare therapeutische Gesichtsmaske (100) nach Anspruch 5, wobei die LED (121) in Silikonmaterial unter Verwendung eines thermoplastischen Materials eingebettet sind, das aus der Gruppe ausgewählt ist, die aus einem thermoplastischen Gummi, TPR, oder einem thermoplastischen Polyurethan, TPU, oder und einem thermoplastischen Elastomer, TPE, besteht.

## Revendications

1. Masque facial thérapeutique réglable (100), dans lequel ledit masque facial thérapeutique réglable (100) recouvre sensiblement un visage humain, comprenant :
un corps de masque (110) ayant une partie intérieure (113) et une partie extérieure (114) ;
une carte de circuit imprimé souple (120) comportant une pluralité de diodes électroluminescentes (DEL) (121) vers la partie intérieure (113) dudit corps de masque (110) ;
une unité de commande (150) couplée à la partie extérieure dudit corps de masque (110) ; et
une feuille de matériau en silicone transparent (130) recouvrant ladite carte de circuit imprimé souple (120), dans lequel ladite pluralité de DEL (121) émettent différentes couleurs et intensités de lumière sur la base d'entrées provenant de ladite unité de commande (150), dans lequel ledit corps de masque (110) présente des découpes pour les yeux, les narines et la bouche pour fixer confortablement ledit masque (100) sur un visage humain, dans lequel ladite carte de circuit imprimé souple (120) est fixée entre ledit corps de masque (110) et ladite feuille de matériau en silicone transparent (130) vers la partie intérieure (113) dudit corps de masque (110) ;
**caractérisé en ce que :**
les bords (131, 132) de ladite feuille de matériau en silicone transparent (130) près de la zone des yeux d'un utilisateur sont rendus opaques pour empêcher la lumière projetée de pénétrer dans les yeux d'un utilisateur.

2. Masque facial thérapeutique réglable (100) selon la revendication 1, dans lequel ledit corps de masque (110) est constitué d'un matériau rigide.

3. Masque facial thérapeutique réglable (100) selon la revendication 1, dans lequel ladite unité de commande (150) est configurée pour commander la couleur et l'intensité de la lumière provenant desdites DEL (121) via des boutons comportant un bouton-poussoir (151) monté vers la partie extérieure (114) dudit corps de masque (110).

4. Masque facial thérapeutique réglable selon la revendication 1, dans lequel l'unité de commande (150) et la pluralité de DEL (121) sont alimentées par un ensemble de batteries rechargeables (160) montées sur ledit corps de masque (110).

5. Masque facial thérapeutique réglable (100) selon la revendication 1, dans lequel lesdites DEL (121) sont des DEL de qualité médicale personnalisées à technologie de montage en surface, SMT, à double puce/spectre.

6. Masque facial thérapeutique réglable (100) selon la revendication 1, dans lequel une sangle extensible (190) est fixée de manière détachable audit masque facial (100) pour fixer de manière réglable ledit masque facial (100) sur la zone cutanée faciale d'un utilisateur.

7. Masque facial thérapeutique réglable (100) selon la revendication 1, dans lequel ladite unité de commande (150) présente un module de communication adapté pour permettre la communication entre un dispositif de communication électronique numérique externe et ledit masque facial thérapeutique réglable (100).

8. Masque facial thérapeutique réglable (100) selon la revendication 7, dans lequel un utilisateur peut régler des couleurs et intensités de ladite pluralité de DEL (121) à l'aide d'une application logicielle installée sur ledit dispositif externe sans retirer ledit masque facial (100).

9. Masque facial thérapeutique réglable (100) selon la revendication 7, dans lequel le masque facial est adapté pour utiliser des spectres de lumière DEL à longueurs d'onde multiples pour stimuler le développement de collagène.

10. Masque facial thérapeutique réglable (100) selon la revendication 9, dans lequel ledit spectre à longueurs d'onde multiples comporte une lumière ayant la longueur d'onde sélectionnée dans le groupe consistant en 880 nm, 660 nm, 630 nm et 605 nm.

11. Masque facial thérapeutique réglable (100) selon la revendication 5, dans lequel les DEL (121) sont encastrées dans un matériau de silicium à l'aide d'un matériau thermoplastique choisi dans le groupe consistant en un caoutchouc thermoplastique, TPR, ou un polyuréthane thermoplastique, TPU, ou et un élastomère thermoplastique, TPE.
